# FASCICULE DE BREVET EUROPEEN

(11) **EP 0 481 855 B1**
(45) Date de publication et mention de la délivrance du brevet: **05.04.1995**
(21) Numéro de dépôt: 91402719.8
(22) Date de dépôt: 11.10.1991
(51) Int. Cl.: A61F 2/32

(54) **Prothèse d'articulation sphérique**
Kugelgelenkprothese
Ball joint prosthesis

(30) Priorité: 16.10.1990 FR 9013017; 07.12.1990 FR 9015345; 11.01.1991 FR 9100279
(43) Date de publication de la demande: 22.04.1992
(73) Titulaire: Bouvet, Jean-Claude, F-91590 La Ferte Alais (FR)
(72) Inventeur: Bouvet, Jean-Claude, F-91590 La Ferte Alais (FR)
(74) Mandataire: Flavenot, Bernard

(56) Documents cités:
- EP-A- 0 066 092
- EP-A- 0 303 195
- DE-A- 2 024 583
- FR-A- 2 060 179
- FR-A- 2 357 235
- US-A- 3 683 421

## Description

La présente invention concerne les prothèses rotuliennes telles que celles qui sont implantées dans le corps humain pour restaurer des articulations détérioriées, par exemple lors d'une maladie ou d'un accident, notamment celles raccordant les os des membres, bras et jambes, au tronc, mais aussi celles raccordant les os des différentes parties de ces membres, par exemple l'articulation de la hanche, celle du genou, etc.

Les chirurgiens sont en effet de plus en plus fréquemment amenés à pratiquer des opérations qui consistent à implanter des prothèses rotuliennes, dont les plus courantes sont celles de la hanche et du genou.

Toute rotule comporte essentiellement une partie mâle généralement constituée par une portion de sphère et une partie femelle en forme de cupule complémentaire de la portion de sphère et de même rayon.

Dans l'exemple d'une prothèse rotulienne coxo-fémorale, la portion de sphère est montée par tous moyens sur une patte de fixation destinée à être implantée dans le fémur, et la cupule est apte à être implantée dans l'os du bassin, la portion de sphère étant maintenue dans la cupule par tous moyens de rétention et étant apte à s'y mouvoir en rotation de façon à permettre les mêmes mouvements de la jambe par rapport au bassin que ceux qui sont normalement possibles avec une articulation saine.

Dans toutes les prothèses rotuliennes connues à ce jour, la cupule dans laquelle est logée en rotation la tête sphérique mâle de la rotule est généralement constituée d'un matériau moins dur que celui dans lequel est réalisée cette tête sphérique. Il s'en suit que le frottement des deux matériaux l'un contre l'autre au cours de la rotation de la tête sphérique dans la cupule produit des particules d'usure du matériau le plus tendre.

Ces particules sont reçues par l'organise vivant dans lequel est implantée la prothèse, par exemple le corps humain, comme des corps étrangers, et cet organisme vivant réagit en augmentant le développement du nombre de ses globules blancs dits "macrophages" et en les guidant vers ces corps étrangers, pour les éliminer. Or, la nature du matériau constituant les particules d'usure de la prothèse ne permet pas aux macrophages de les digérer et les particules ont tendance à migrer dans le corps, dans l'environnement de la prothèse, notamment le long de la patte de fixation. Pour combattre ces corps étrangers, les macrophages s'agglutinent autour d'eux en colonies de plus en plus denses. La prothèse elle-même devient un corps étranger à détruire et les macrophages ont tendance à se concentrer autour d'elle, entre elle et la partie d'os sur laquelle ou dans laquelle elle est fixée. Ces agrégats de macrophages entraînent petit à petit le descellement de la prothèse.

Pour tenter de pallier cet inconvénient, on a réalisé une prothèse rotulienne qui comporte une coupelle définissant un logement en creux hémisphérique d'une première valeur de rayon de courbure, une portion de sphère ayant une deuxième valeur de rayon de courbure, cette deuxième valeur étant inférieure à la première valeur, une pluralité de billes sphériques, les billes sphériques ayant un diamètre d'une troisième valeur égale à la différence entre les prière et deuxième valeurs de rayon de courbure, la portion de sphère étant située dans le logement de la coupelle de façon que son centre et celui du logement soient confondus pour définir, entre elle et le logement, un espace hémisphérique, les billes étant disposées dans l'espace hémisphérique entre la paroi du logement et la paroi de la portion de sphère, et à leur contact, et des moyens pour retenir les billes dans l'espace hemisphérique. Une telle prothèse est connue du document FR-A-2 060 179.

La structure de prothèse rotulienne décrite ci-dessus donne de bons résultats. Cependant, la présente invention a pour but de réaliser une prothèse rotulienne qui permette de réduire encore plus la quantité produite de particules d'usure et de favoriser la rotation des billes entre elles et par rapport aux surfaces qui les entourent.

Plus précisément, la présente invention a pour objet une prothèse rotulienne comportant :
au moins une coupelle définissant un logement en creux hémisphérique d'une prière valeur de rayon de courbure,
au moins une portion de sphère ayant une deuxième valeur de rayon de courbure, cette dite deuxième valeur étant inférieure à ladite prière valeur,
une pluralité de billes sphériques ayant un diamètre d'une troisième valeur égale à la différence entre lesdites prière et deuxième valeurs de rayon de courbure, ladite portion de sphère étant située dans ledit logement de la coupelle de façon que son centre et celui du logement soient confondus pour définir, entre elle et ledit logement, un espace hémisphérique, lesdites billes étant disposées dans ledit espace hémisphérique entre la paroi dudit logement et la paroi de ladite portion de sphère, et
des moyens pour retenir lesdites billes dans ledit espace hémisphérique,
caractérisée par le fait qu'elle comporte en outre une cavité réalisée dans ladite coupelle, ladite cavité comportant une ouverture circulaire définie sensiblement dans la paroi dudit logement hémisphérique et sensiblement centrée sur l'axe de révolution dudit logement, ladite ouverture reliant ledit espace hémisphérique et ladite cavité.

D'autres caractéristiques et avantages de la présente invention apparaîtront au cours de la description suivante donnée en regard des dessins annexés à titre illustratif, mais nullement limitatif, dans lesquels :
Les figures 1 et 3 représentent deux vues en coupe, respectivememnt référencées I-I sur la figure 3 et III-III sur la figure 1, d'un mode de réalisation d'une prothèse rotulienne comportant le perfectionnement selon l'invention,
La figure 2 représente, à une plus grande échelle, un détail du mode de réalisation de la prothèse rotulienne selon la figure 1,
La figure 4 représente, vu en coupe et partiellement écorché, un mode de réalisation d'une prothèse rotulienne comportant le perfectionnement à la réalisation selon les figures 1 à 3, et
La figure 5 représente, vu en coupe et partiellement écorché, un autre mode de réalisation d'une prothèse rotulienne selon l'invention.

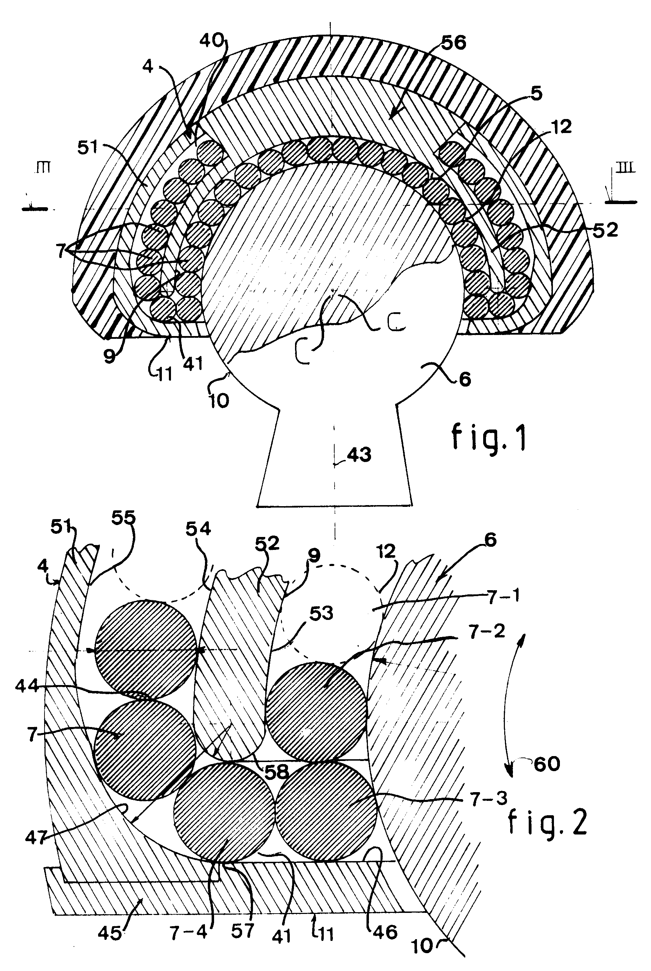

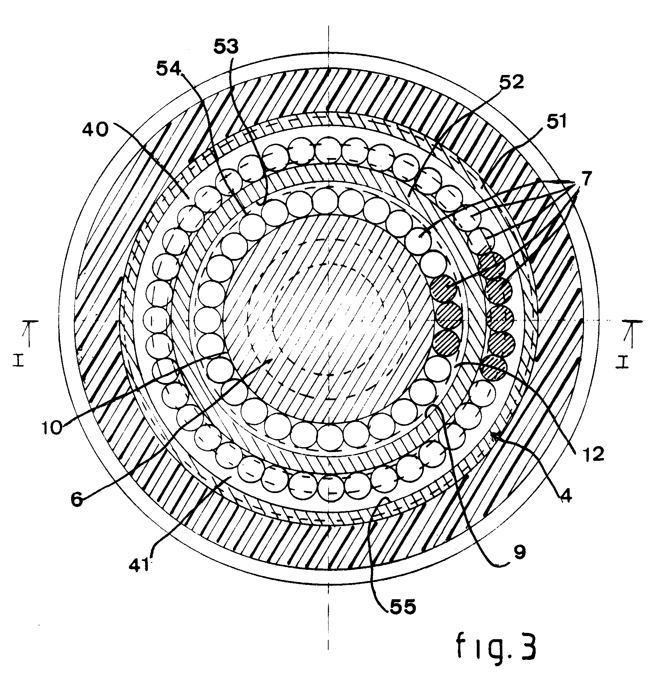

La prothèse rotulienne dont un mode de réalisation est illustré sur les figures 1 à 3 comporte au moins une coupelle 4 définissant un logement en creux sensiblement hémisphérique 5 d'un rayon de courbure "R" ayant une prière valeur, et au moins une portion de sphère 6 ayant une deuxième valeur de rayon de courbure "r", la deuxième valeur "r" étant inférieure à la prière valeur "R".

La portion de sphère 6 est située dans le logement 5 de la coupelle 4 de façon que son centre "c" soit confondu avec celui "C" du logement, pour définir entre elle et ce logement un espace hémisphérique 12, des billes 7 étant disposées dans cet espace hémisphérique entre la paroi 9 du logement 5 et la paroi 10 de la portion de sphère 6.

Les billes 7 ont un diamètre d'une troisiéme valeur égale à la différence entre lesdites première et deuxième valeurs de rayon de courbure de façon qu'elles soient au contact de la paroi 9 du logement et de la paroi 10 de la portion de sphère.

Selon une caractéristique importante, la prothèse comporte en outre une cavité 40 réalisée dans la coupelle 4. Cette cavité 40 comporte une ouverture circulaire 41 la reliant à l'espace hémisphérique 12 définie sensiblement dans la paroi 9 du logement 5, avantageusement au niveau de son fond, et sensiblement centrée sur l'axe de révolution 43 de ce logement.

Dans le mode de réalisation illustré, l'espace hémisphérique 12 et la cavité 40 sont remplis de billes 7 de façon que ces dernières y soient réparties sur une seule nappe et qu'elles soient au contact les unes des autres en au moins un point commun 44.

Selon une caractéristique avantageuse, la largeur de l'ouverture circulaire 41 est sensiblement égale au diamètre des billes 7 en contact avec la paroi du logement et la portion de sphère.

Dans une réalisation possible, l'épaisseur de la cavité 40 est égale au diamètre des billes 7. Cependant, pour favoriser le roulement de ces billes, cette épaisseur peut avantageusement être très légèrement supérieure à leur diamètre, comme représenté sur les figures.

Bien entendu, a prothèse comporte des moyens 11 pour retenir les billes dans l'espace hémisphérique 12 et, selon une caractéristique du perfectionnement, les moyens 11 sont constitués par une bague 45 solidaire de la coupelle 4. La bague 45 a une configuration qui lui permet de border l'ouverture 41, en même temps que sa surface 46 tournée vers l'espace hémisphérique 12 forme une continuité avec la surface concave 47, la plus éloignée par rapport au logement 5, de la cavité 40.

Dans un mode de réalisation avantageux, la cavité 40 est délimitée par deux pièces 51, 52 en forme de coupe placées l'une dans l'autre et formant, avec les moyens décrits ci-après permettant leur association, la coupelle 4.

La coupe intérieure 52 est délimitée par deux prière 53 et seconde 54 surfaces respectivement concave et convexe, toutes deux essentiellement hémisphériques et concentriques, la prière surface concave 53 formant une partie de la paroi 9 du logement en creux 5.

L'autre coupe 51 définit une troisième surface hémisphérique concave 55 ayant un rayon de courbure au moins égal, sinon très légèrement supérieur, à la somme du rayon de courbure de la seconde surface convexe 54 de la coupe intérieure 52 et du diamètre des billes 7 en contact avec les parois du logement et de la portion de sphère.

Comme mentionné ci-avant, la coupelle 4 comporte des moyens 56 pour associer les deux coupes 51, 52 de façon que les trois prière 53, deuxième 54 et troisième 55 surfaces soient concentriques et que le bord 57 de la troisième surface, au niveau de l'ouverture circulaire 41 de la cavité 40, entoure la portion de paroi 58 reliant les deux prière 53 et deuxième 54 surfaces en restant éloignée de cette portion de paroi d'une distance au moins égale au diamètre des billes 7. Les moyens 56 pour associer les deux coupes 51, 52 sont avantageusement constitués par au moins une entretoise solidaire des deuxième et troisième surfaces. Cette entretoise, de préférence dépourvue de portions de contour aiguës, est par exemple de forme cylindrique de révolution.

La prothèse décrite ci-dessus fonctionne de la façon suivante:
On suppose par exemple que, par référence aux deux figures 1 et 2, la portion de sphère 6 subit une rotation senestrorsum 60. Dans ce cas, elle entraîne les billes 7 situées dans le logement 5, comme les billes 7-1, 7-2, ..., dans des rotations dextrorsum, par lesquelles elles glissent sur la surface 53 en tendant à pousser les billes 7-3, 7-4, ... qui sont dans la cavité 40, ou à son entrée, pour réalimenter l'espace hémisphérique 12 par la partie d'ouverture 41 opposée à celle par laquelle les billes 7 ont été envoyées dans la cavité 40.

La rotation de la portion de sphère 6 est ainsi facilitée par le fait que certaines des billes ne sont plus obligées de frotter, dans une rotation sur elles-mêmes, contre le fond du logement en creux.

Ce résultat est le même quelle que soit la rotation de la portion de sphère 6 par rapport à la coupelle 4, puisque l'ouverture 41 est circulaire et que les billes éjectées de l'espace 12 vers la cavité 40 sont automatiquement remplacées par des billes provenant de la cavité 40 et entrant dans l'espace 12 par la partie d'ouverture opposée à celle par laquelle les billes éjectées avaient quitté cet espace.

Afin de réduire encore plus le frottement des billes, notamment dans la cavité 40, celle-ci a une épaisseur très légèrement supérieure au diamètre des billes, comme représenté sur les figures sur lesquelles cette différence a été exagérée à dessein.

Dans ces conditions, la rotation des deux parties mâle et femelle de la prothèse rotulienne l'une par rapport à l'autre est obtenue avec un minimum de frottements, ce qui entraîne une importante réduction de la quantité produite de particules d'usure des matériaux en contact.

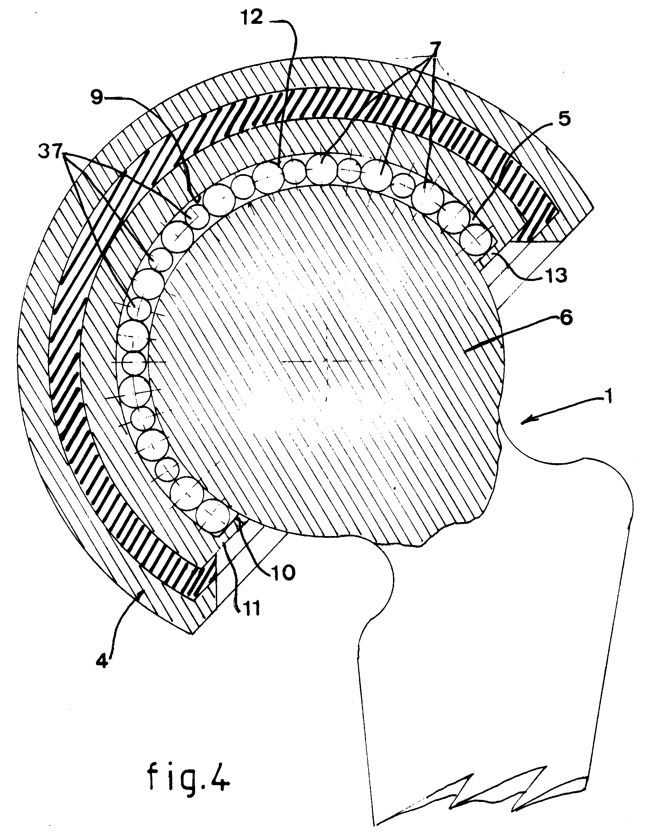

La figure 4 représente un perfectionnement à la réalisation illustrée sur les figures 1 à 3.

Dans le mode de réalisation illustré de ce perfectionnement, les billes emplissant le logement 5 et la cavité 40 (le logement 5 étant seul représenté par souci de simplification du dessin) sont constituées par une pluralité de premières billes sphériques 7 qui ont un diamètre "D" d'une valeur égale à la différence entre les première "R" et deuxième "r" valeurs de rayon de courbure et par une autre pluralité de secondes billes 37, ces secondes billes 37 ayant une quatrième valeur de diamètre "d" inférieure à la valeur "D" du diamètre des premières billes 7.

Avantageusement, le nombre de secondes billes 37 est inférieur à celui des prières billes 7, de façon que la partie sphérique 6 de la prothèse soit bien supportée par les prières billes 7 de diamètre "D", alors que, en se répartissant et s'intercalant entre les billes 7, les secondes billes 37 de diamètre "d" permettent d'améliorer la rotation de ces prières billes. En effet, ces secondes billes 37 jouent en quelque sorte le rôle d'entretoises entre les billes 7, mais avec l'avantage que ces entretoises peuvent pivoter sur elles-mêmes.

Le Demandeur a réalisé des prototypes de prothèse rotulienne selon l'invention qui ont donné satisfaction, dans lesquels le pourcentage de secondes billes 37 par rapport au nombre de prières billes 7 était de l'ordre de soixante à soixante-dix pour cent. Le logement 5 et la cavité 40 étaient entièrement remplis de billes 7 et 37 et les billes 37 de plus faible diamètre occupaient donc sensiblement trente-cinq pour cent du volume.

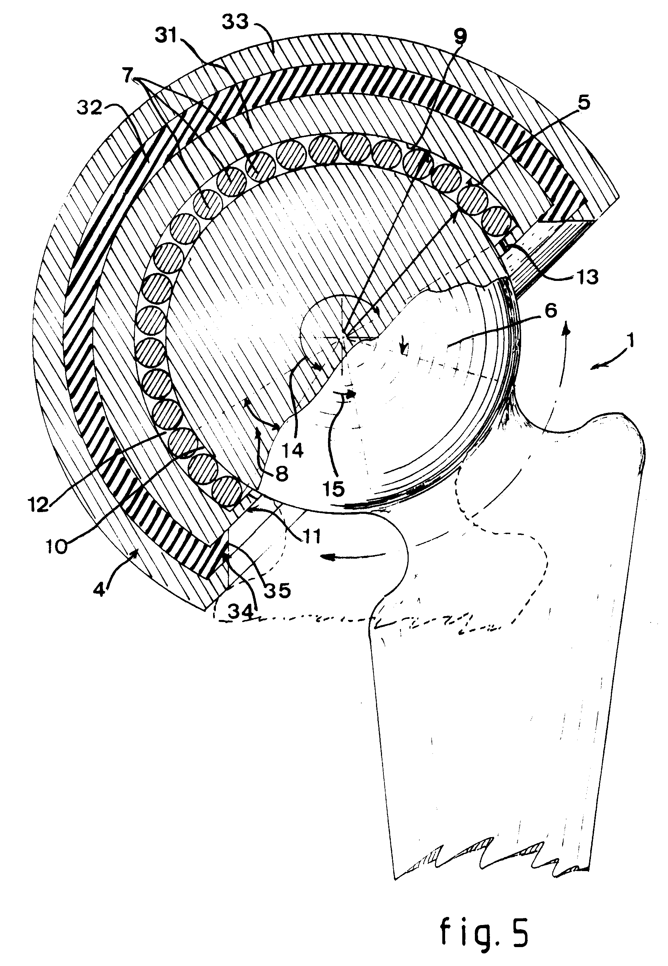

Selon une autre caractéristique avantageuse additionnelle, comme illustré sur la figure 5 sur laquelle, comme pour l'exemple précédent par simple souci de simplification du dessin, la cavité 40 n'a pas été représentée, la coupelle 4 est constituée par un empilement d'au moins trois calottes 31, 32 et 33 les unes dans les autres, les deux calottes intérieure 31 et extérieure 33 étant réalisées en un matériau rigide, la calotte intermédiaire 32 étant réalisée en un matériau présentant une certaine élasticité de déformation, tel qu'un élastomère ou analogue.

Par contre, la portion de sphère 6, les billes 7, 37 et la partie de coupelle 4 au contact des billes, par exemple la calotte intérieure 31, sont réalisées en un même matériau tel que, par exemple, un matériau à base de titane ou de mousse de titane.

A cet empilement de trois calottes 31-33, sont associés des moyens 34 pour maintenir les trois calottes solidaires les unes des autres et éviter, par exemple, qu'elles ne pivotent les unes dans les autres ou qu'elles ne se désunissent. Ces moyens 34 sont très schématiquement représentés, sur la figure 5, par des ergots 35 ou analogue.

Il est en outre avantageux que la valeur de l'angle au centre 14 du logement 5, comme illustré sur la figure 5, soit supérieure à 180 degrés, par exemple de façon que la portion 8 allant au-delà de l'angle plat, de cet angle au centre 14, contienne au moins une bille 7 de diamètre "D" .

Bien évidemment, la valeur de l'angle au centre 15 de la portion de sphère 6 est supérieure à la valeur de l'angle au centre 14 du logement en creux pour que, comme dans toutes les prothèses rotuliennes et en particulier les prothèses coxo-fémorales, le mouvement de rotation de la partie mâle dans la partie femelle puisse avoir l'amplitude nécessairre pour la restitution des mouvements de l'articulation ainsi restaurée.

## Revendications

1. Prothèse rotulienne comportant :
au moins une coupelle (4) définissant un logement en creux hémisphérique (5) d'une prière valeur de rayon de courbure,
au moins une portion de sphère (6) ayant une deuxième valeur de rayon de courbure, cette dite deuxième valeur étant inférieure à ladite prière valeur,
une pluralité de premières billes sphériques (7) ayant un diamètre d'une troisième valeur égale à la différence entre lesdites prière et deuxième valeurs de rayon de courbure, ladite portion de sphère étant située dans ledit logement de la coupelle de façon que son centre et celui du logement soient confondus pour définir, entre elle et ledit logement, un espace hémisphérique (12), lesdites prières billes étant disposées dans ledit espace hémisphérique entre la paroi (9) dudit logement et la paroi (10) de ladite portion de sphère, et
des moyens (11) pour retenir lesdites billes dans ledit espace hémisphérique (12),
caractérisée par le fait qu'elle comporte en outre une cavité (40) réalisée dans ladite coupelle (4), ladite cavité comportant une ouverture circulaire (41) définie sensiblement dans la paroi (9) dudit logement (5) et sensiblement centrée sur l'axe de révolution (43) dudit logement, ladite ouverture reliant ledit espace hémisphérique (12) et ladite cavité (40).

2. Prothèse selon la revendication 1, caractérisée par le fait que la largeur de ladite ouverture (41) est sensiblement égale au diamètre desdites prières billes (7).

3. Prothèse selon la revendication 2, caractérisée par le fait que l'épaisseur de ladite cavité (40) est légèrement supérieure audit diamètre desdites prières billes (7).

4. Prothèse selon l'une des revendications précédentes, caractérisée par le fait que les moyens (11) pour retenir lesdites billes dans ledit espace hémisphérique (12) sont constitués par une bague (45) solidaire de ladite coupelle (4), ladite bague délimitant l'un des bords de ladite ouverture (41), la surface (46) de ladite bague tournée vers ledit espace hémisphérique (12) formant une continuité avec une surface (47,55) de ladite cavité.

5. Prothèse selon l'une des revendications précédentes, caractérisée par le fait que ladite coupelle (4) est formée de deux pièces (51,52) en forme de coupes, l'une étant placée sensiblement dans l'autre, la coupe intérieure (52) étant délimitée par deux première (53) et seconde (54) surfaces respectivement concave et convexe, toutes deux hémisphériques et concentriques, la première surface concave (53) formant la paroi (9) du logement en creux (5), l'autre coupe (51) définissant une troisième surface hémisphérique (55) concave ayant un rayon de courbure au moins égal à la somme du rayon de courbure de la deuxième surface convexe (54) et du diamètre desdites premières billes (7), et de moyens (56) pour associer les deux dites coupes de façon que les trois première, deuxième et troisième surfaces soient concentriques et que le bord de la troisième surface entoure la portion de paroi (58) reliant les deux première (53) et deuxième (54) surfaces à une distance égale au moins au diamètre desdites premières billes.

6. Prothèse selon la revendication 5, caractérisée par le fait que les moyens (56) pour associer les deux coupes sont constitués par au fins une entretoise solidaire des deuxième (54) et troisième (55) surfaces.

7. Prothèse selon l'une des revendications précédentes, caractérisée par le fait qu'il comporte en outre une pluralité de secondes billes (37), lesdites secondes billes ayant une quatrième valeur de diamètre inférieure à ladite troisième valeur de diamètre des premières billes (7) et étant disposées dans ledit espace hémisphérique et ladite cavité.

8. Prothèse selon la revendication 7, caractérisée par le fait que le nombre de secondes billes (37) est inférieur au nombre de permières billes (7).

9. Prothèse selon l'une des revendications précédentes, caractérisée par le fait que ledit logement en creux (5) a une valeur d'angle au centre (14) supérieure à 180 degrés.

10. Prothèse selon la revendication 9, caractérisée par le fait que la valeur de l'angle au centre (15) de la portion de sphère (6) est supérieure à la valeur de l'angle au centre (14) dudit logement en creux (5).

11. Prothèse selon la revendication 10, caractérisée par le fait que la valeur de l'angle au centre (14) dudit logement en creux (5) est déterminée de façon que la portion (8), au-delà de l'angle plat, dudit angle au centre du logement en creux, contienne au moins une première bille (7).

12. Prothèse selon l'une des revendications précédentes, caractérisée par le fait que ladite coupelle (4) est constituée par un empilement d'au moins trois calottes (31,32,33) les unes dans les autres, les deux calottes intérieure (31) et extérieure (33) étant réalisées en un matériau rigide, la calotte intermédiaire (32) étant réalisée en un matériau présentant une élasticité de déformation.

## Claims

1. A ball-joint prosthesis comprising:
at least one cup (4) defining a hemispherical hollow housing (5) having a radius of curvature of a first value;
at least one portion of a sphere (6) having a radius of a second curvature value, said second value being less than said first value;
a plurality of first spherical beads (7) having a diameter of a third value equal to the difference between said first and second values for radius of curvature, said portion of a sphere being situated in said housing of the cup in such a manner that its center coincides with the center of the housing so as to define a hemispherical space (12) between the housing and the portion of a sphere, said first beads being disposed in said hemispherical space between the wall (9) of said housing and the wall (10) of said portion of a sphere; and
means (11) for retaining said beads in said hemispherical space (12);
the prosthesis being characterized by the fact that it further comprises a cavity (40) formed in said cup (4), said cavity including a circular opening (41) defined substantially in the wall (9) of said housing (5) and substantially centered on the axis of symmetry (43) of said housing, said opening interconnecting said hemispherical space (12) and said cavity (40).

2. A prosthesis according to claim 1, characterized by the fact that the width of said opening (41) is substantially equal to the diameter of said first beads (7).

3. A prosthesis according to claim 2, characterized by the fact that the thickness of said cavity (40) is slightly greater than said diameter of said first beads (7).

4. A prosthesis according to any preceding claim, characterized by the fact that the means (11) for retaining said beads in said hemispherical space (12) are constituted by a ring (45) secured to said cup (4), said ring defining one of the edges of said opening (41), the surface (46) of said ring that faces towards said hemispherical space (12) cooperating with a surface (47, 55) of said cavity to form a continuous surface.

5. A prosthesis according to any preceding claim, characterized by the fact that said cup (4) is made up of two cup-shaped parts (51, 52), one being placed substantially within the other, the inner cup (52) being defined by two surfaces, respectively a concave first surface (53) and a convex second surface (54), both surfaces being hemispherical and concentric, the concave first surface (5'') forming the wall (9) of the hollow housing (5), the other cup (51) defining a concave third hemispherical surface (55) having a radius of curvature that is not less than the sum of the radius of curvature of the convex second surface (54) plus the diameter of said first beads (7), and means (56) for associating said two cups in such a manner that the first, second, and third surfaces are concentric and the edge of the third surface surrounds the wall portion (58) interconnecting the first and second surfaces (53, 54) at a distance that is not less than the diameter of said first beads.

6. A prosthesis according to claim 5, characterized by the fact that the means (56) for associating the two cups are constituted by at least one spacer secured to the second and third surfaces (54, 55).

7. A prosthesis according to any preceding claim, characterized by the fact that it further comprises a plurality of second beads (37), the diameter of said second beads having a fourth value that is less than said third value for the diameter of said first beads (7) and being disposed in said hemispherical space and said cavity.

8. A prosthesis according to claim 7, characterized by the fact that the number of second beads (37) is less than the number of first beads (7).

9. A prosthesis according to any preceding claim, characterized by the fact that said hollow housing (5) has an angle at the center (14) of value greater than 180°.

10. A prosthesis according to claim 9, characterized by the fact that the value of the angle at the center (15) of the portion of a sphere (6) is greater than the value of the angle at the center (14) of said hollow housing (5).

11. A prosthesis according to claim 10, characterized by the fact that the value of the angle at the center (14) of said hollow housing (5) is determined in such a manner that the portion (8) of said angle at the center of the hollow housing that extends beyond 180° includes at least one first bead (7).

12. A prosthesis according to any preceding claim, characterized by the fact that said cup (4) is constituted by a stack of at least three spherical caps (31, 32, 33) in one another, the inner and outer caps (31, 33) being made of a rigid material and the intermediate cap (32) being made of a material that deforms resiliently.

## Patentansprüche

1. Kugelgelenkprothese, umfassend:
mindestens eine Kuppel (4), die eine halbkugelige hohle Ausnehmung (5) mit einem ersten Wert des Krümmungsradius begrenzt,
mindestens einen Abschnitt der Kugel (6) mit einem zweiten Wert des Krümmungsradius, welcher zweite Wert kleiner ist als der erste Wert,
eine Mehrzahl von ersten Kugeln (7) mit einem Durchmesser eines dritten Werts gleich der Differenz zwischen dem ersten und dem zweiten Krümmungsradiuswert, welcher Kugelabschnitt sich in der Ausnehmung der Kuppel derart befindet, daß sein Zentrum und dasjenige der Ausnehmung zusammenfallen, um zwischen diesem Kugelabschnitt und der Ausnehmung einen halbkugeligen Raum (12) zu begrenzen, wobei die ersten Kugeln in dem halbkugeligen Raum zwischen der Wandung (9) der Ausnehmung und der Wandung (10) des genannten Kugelabschnitts angeordnet sind, und
Mittel (11) zum Halten der Kugeln in dem halbkugeligen Raum (12),
dadurch gekennzeichnet, daß sie ferner einen Hohlraum (40) umfaßt, der in der Kuppel (4) ausgebildet ist, welcher Hohlraum eine runde Öffnung (41) aufweist, die im wesentlichen in der Wandung (9) der Ausnehmung (5) begrenzt ist und im wesentlichen zentriert ist auf die Umlaufachse (43) der Ausnehmung, welche Öffnung den halbkugeligen Raum (12) und den Hohlraum (40) verbindet.

2. Prothese nach Anspruch 1, dadurch gekennzeichnet, daß die Breite der Öffnung (41) im wesentlichen gleich dem Durchmesser der ersten Kugeln (7) ist.

3. Prothese nach Anspruch (2), dadurch gekennzeichnet, daß die Höhe des Hohlraums (40) etwas größer ist als der Durchmesser der ersten Kugeln (7).

4. Prothese nach einem der vorangehenden Ansprüche, dadurch gekennzeichnet, daß die Mittel (11) zum Halten der Kugeln in dem halbkugeligen Raum (12) von einem Ring (45) gebildet werden, der mit der Kuppel (4) verbunden ist, welcher Ring einen der Ränder der Öffnung (41) begrenzt, wobei die Oberfläche (46) des Rings, der dem halbkugeligen Raum (12) zugekehrt ist, eine Fortsetzung mit einer Oberfläche (47, 55) des Hohlraums bildet.

5. Prothese nach einem der vorangehenden Ansprüche, dadurch gekennzeichnet, daß die Kuppel (4) aus zwei Teilen (51, 52) in Form von Abschnitten ausgebildet ist, wobei der eine im wesentlichen in dem anderen plaziert ist, wobei der innere Abschnitt (52) begrenzt wird von zwei ersten (53) und zweiten (54) Oberflächen, die konkav bzw. konvex sind, beide halbkugelig und konzentrisch, wobei die erste konkave Oberfläche (53) die Wandung (9) der hohlen Ausnehmung (5) bildet, wobei der andere Abschnitt (51) eine dritte halbkugelige konkave Oberfläche (55) begrenzt mit einem Krümmungsradius, der mindestens gleich ist der Krümmungsradien der zweiten konvexen Oberfläche (54) und dem Durchmesser der ersten Kugeln (7) und mit Mitteln (56) zum Verbinden der beiden Abschnitte derart, daß die drei ersten, zweiten und dritten Oberflächen konzentrisch sind, und daß der Rand der dritten Oberfläche den Abschnitt der Wandung (58) umschließt, welche die beiden ersten (53) und zweiten (54) Oberflächen mit einem Abstand gleich mindestens dem Durchmesser der ersten Kugeln verbindet.

6. Prothese nach Anspruch 5, dadurch gekennzeichnet, daß die Mittel (56) zum Fügen der beiden Abschnitte von mindestens einem Distanzstück gebildet werden, das mit der zweiten (54) und dritten (55) Oberfläche verbunden ist.

7. Prothese nach einem der vorangehenden Ansprüche, dadurch gekennzeichnet, daß sie ferner eine Mehrzahl von zweiten Kugeln (37) umfaßt, welche zweiten Kugeln einen vierten Durchmesserwert besitzen, der kleiner ist als der dritte Wert des Durchmessers der ersten Kugeln (7) und die in dem halbkugeligen Raum und dem Hohlraum angeordnet sind.

8. Prothese nach Anspruch 7, dadurch gekennzeichnet, daß die Anzahl der zweiten Kugeln (37) geringer ist als die Anzahl der ersten Kugeln (7).

9. Prothese nach einem der vorangehenden Ansprüche, dadurch gekennzeichnet, daß die hohle Ausnehmung (5) einen Wert des Zentrumswinkels (14) oberhalb 180° aufweist.

10. Prothese nach Anspruch 9, dadurch gekennzeichnet, daß der Wert des Zentrumswinkels (15) des Abschnitts der Kugel (6) größer ist als der Wert des Zentrumswinkels (14) der hohlen Ausnehmung (5).

11. Prothese nach Anspruch 10, dadurch gekennzeichnet, daß der Wert des Zentrumswinkels (14) der hohlen Ausnehmung (5) derart bestimmt wird, daß der Abschnitt (8) jenseits des flachen Winkels des Zentrumswinkels der hohlen Ausnehmung mindestens eine erste Kugel (7) enthält.

12. Prothese nach einem der vorangehenden Ansprüche, dadurch gekennzeichnet, daß die Kuppel (4) aus einer Schichtung von mindestens drei Kalotten (31, 32, 33) ineinander besteht, wobei die beiden inneren (31) und äußeren (33) Kalotten aus einem starren Material hergestellt sind, während die dazwischen liegende Kalotte (32) aus einem Material hergestellt ist, das eine Deformationselastizität aufweist.
